# EUROPÄISCHE PATENTANMELDUNG

(11) **EP 0 779 278 A2**
(43) Veröffentlichungstag der Anmeldung: **18.06.1997**
(21) Anmeldenummer: 96115996.9
(22) Anmeldetag: 05.10.1996
(51) Int. Cl.: C07D 229/00, C08G 18/79, C08G 18/80, C09D 175/00

(54) **Uretdiongruppenhaltige Polyadditionsprodukte sowie deren Verwendung**

(30) Priorität: 14.12.1995 DE 19546750
(71) Anmelder: HÜLS AKTIENGESELLSCHAFT, 45764 Marl (DE)
(72) Erfinder: Gras, Rainer, Dr., 44879 Bochum (DE); Schmitt, Felix, Dr., 45701 Herten (DE); Wolf, Elmar, Dr., 45661 Recklinghausen (DE)

(57) **Zusammenfassung**

Uretdiongruppenhaltige Polyadditionsprodukte der allgemeinen Formel I, erhältlich durch Reaktion des Isophorondiisocyanat-Uretdions (IPDI-Uretdion) mit di-sekundären Diaminen 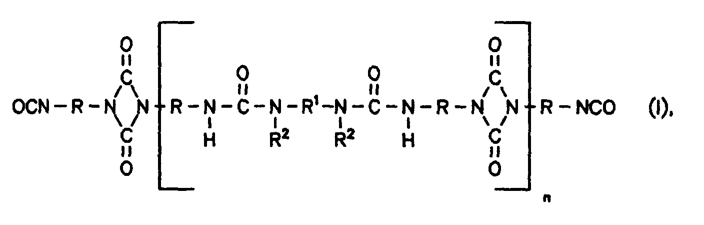 wobei
R der Formel II entspricht 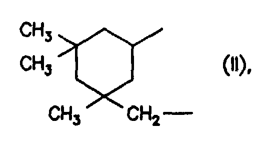 und
R¹ gleich R oder einen cycloaliphatischen oder aliphatischen, linearen oder verzweigten Alkylenrest mit 2 - 16 C-Atomen,
R² einen cycloaliphatischen oder aliphatischen Kohlenwasserstoffrest mit 1 - 12 C-Atomen oder einen Rest der Formel III 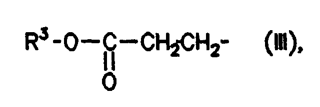
R³, R⁴, und R⁵ gleiche oder verschiedene Kohlenwasserstoffreste mit 1 - 14 C-Atomen und n gleich 1 - 15 bedeuten.

## Beschreibung

Gegenstand der vorliegenden Erfindung sind uretdiongruppenhaltige Polyadditionsprodukte mit freien, partiell oder ganz blockierten Isocyanatgruppen, ein Verfahren zu ihrer Herstellung sowie die Verwendung in Polyurethan-Pulverlacksystemen.

Uretdiongruppenhaltige Diisocyanat-Diol-Addukte sind aus der DE-OS 30 30 572 bekannt. Ihre Verwendung in Polyurethan-Pulverlacken wird in der DE-OS 30 30 554 beschrieben. Ein Nachteil dieser Verbindungen besteht in der langen Reaktionszeit, die für ihre Herstellung benötigt wird (0,5 - 5 h; S. 5, Zeile 11, DE-OS 30 30 572), da man unter diesen Bedingungen bereits mit einer Dissoziation des Uretdionringes rechnen muß.

Aufgabe der vorliegenden Erfindung war es deshalb, uretdiongruppenhaltige Polyadditionsprodukte zur Verfügung zu stellen, die den Nachteil der bekannten uretdiongruppenhaltigen Polyadditionsverbindungen nicht aufweisen.

Diese Aufgabe wurde durch die Lehre gemäß den Patentansprüchen gelöst.

Gegenstand der vorliegenden Erfindung sind somit uretdiongruppenhaltige Polyadditionsprodukte der allgemeinen Formel I, erhältlich durch Reaktion des Isophorondiisocyanat-Uretdions (IPDI-Uretdion) mit di-sekundären Diaminen 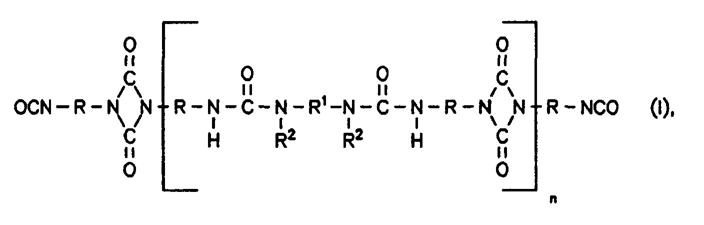 wobei
R der Formel II entspricht 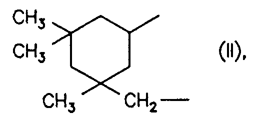 und
R¹ gleich R oder einen cycloaliphatischen oder aliphatischen, linearen oder verzweigten Alkylenrest mit 2 - 16 C-Atomen,
R² einen cycloaliphatischen oder aliphatischen Kohlenwasserstoffrest mit 1 - 12 C-Atomen oder einen Rest der Formel III 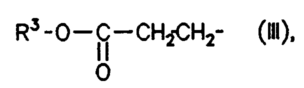
R³, R⁴, und R⁵ gleiche oder verschiedene Kohlenwasserstoffreste mit 1 - 14 C-Atomen und n gleich 1 - 15 bedeuten.

Die erfindungsgemäßen Verbindungen weisen einen Gehalt an freien NCO-Gruppen von 0,5 - 6 % Gew., vorzugsweise 1 - 3 %, einen Uretdiongruppengehalt von 1,3 - 1,7 mmol/g sowie einen Gehalt an (reversibel wie irreversibel) blockierten NCO-Gruppen von 0 - 1,7 mmol/g auf. Ihr Schmelzpunkt variiert in einem Bereich von 60 - 170 °C. Sie eignen sich in hervorragender weise zur Herstellung von Polyurethan-Pulverbeschichtungen.

Ein weiterer Gegenstand der vorliegenden Erfindung ist ein Verfahren zur Herstellung der erfindungsgemäßen Verbindungen entsprechend der folgenden Reaktionsgleichung:

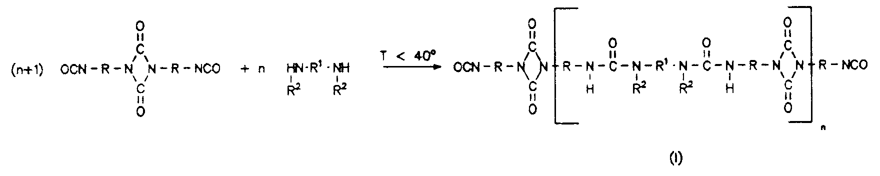

Bei dem erfindungsgemäß einzusetzenden Uretdion handelt es sich um das Isophorondiisocyanat-Uretdion (IPDI-Uretdion), wie es in den DE-OSS 30 30 513 und 37 39 549 beschrieben wird, mit einem freien NCO-Gehalt von 17 - 18 %, d. h. daß mehr oder minder hohe Anteile an Polyuretdion des IPDI im Reaktionsprodukt vorliegen müssen. Der Monomergehalt liegt bei ≤ 1 %. Der Gesamt-NCO-Gehalt des IPDI-Uretdions nach dem Erhitzen auf 180 - 200 °C (0,5 h) beträgt 31,5 - 37,8 %.

Die erfindungsgemäß einzusetzenden Diamine sind di-sekundäre Diamine, die im Handel erhältlich sind, oder in zwei Stufen hergestellt werden, wobei in der 1. Stufe ein (cyclo)aliphatisches Diamin mit zwei primären NH₂-Gruppen mit einem Aldehyd oder Keton zur Schiffschen Base kondensiert wird und in der 2. Stufe die Hydrierung der Schiffschen Base erfolgt. Für die Kondensation zur Schiffschen Base kommen grundsätzlich alle (cyclo)aliphatischen Diamine, wie z. B. Ethylendiamin, 1,2-Diaminopropan, 2-Methyl-pentamethylendiamin, Hexamethylendiamin, 2,2,4(2,4,4)-Trimethylhexamethylendiamin, Isophorondiamin (IPD), 1,2-Diaminocyclohexan sowie 1,3-Bis(aminomethyl)benzol in Frage. Für die Herstellung der Schiffschen Base einzusetzende Carbonylverbindung kommen grundsätzlich alle (cyclo)aliphatischen Aldehyde und Ketone in Frage, bevorzugt werden jedoch Isobutyraldehyd, 2-Ethylhexanal, Methylethylketon, Methylisobutylketon, Diisobutylketon, Cyclohexanon und 3,5,5-Trimethylcyclohexanon eingesetzt. Eine besonders vorteilhafte Variante des erfindungsgemäßen Verfahrens besteht darin, di-sekundäre Diamine einzusetzen, wie sie durch Reaktion von di-primären Diaminen mit Acrylsäureestern, wie z. B. Acrylsäuremethyl-, -ethyl-, -butyl-, 2-ethylhexylester, erhalten werden. Die Umsetzung des Diamins mit dem Acrylsäureester erfolgt bei 60 - 80 °C im Molverhältnis 1:2.

Manchmal hat es sich als zweckmäßig erwiesen, die freien NCO-Gruppen der uretdiongruppenhaltigen Polyadditionsprodukte zu blockieren. Für den Fall, daß die NCO-Gruppen irreversibel blockiert werden, d. h. beim Einbrennvorgang wird das Blockierungsmittel nicht abgespalten, werden die freien NCO-Gruppen des Uretdion-Diamin-Adduktes mit Monoalkoholen, wie z. B. Methanol, Ethanol, Butanol, 2-Ethylhexanol, oder sekundären Monoaminen, wie z. B. Dibutylamin, Di-2-ethylhexylamin, Methylcyclohexylamin, umgesetzt. Für die reversible Blockierung der freien NCO-Gruppen kommen Ketoxime, wie z. B. Acetonoxim, Methylethylketoxim, Acetophenonoxim und Cyclohexanonoxim, zum Einsatz.

Die erfindungsgemäßen uretdiongruppenhaltigen Polyadditionsverbindungen werden nach dem nunmehr erläuterten Verfahren hergestellt. Die Herstellung erfolgt so, daß n+1 mol IPDI-Uretdion mit n mol di-sekundärem Diamin zur Reaktion gebracht werden und dabei die Temperatur nicht über 40 °C steigt. Erfindungsgemäß können die freien NCO-Gruppen vor oder nach der Reaktion mit den di-sekundären Diaminen mit Blockierungsmitteln zur Reaktion gebracht werden.

Die Umsetzung kann jeweils mit oder ohne Lösemittel erfolgen. Im Falle des Einsatzes eines Lösemittels kann dies ausgewählt werden aus der Gruppe aromatischer Kohlenwasserstoffe, Ester oder Ketone, wie z. B. Toluol, Ethyl- oder Butylacetat, Aceton, Methylethylketon, Methylisobutylketon, sowie beliebige Gemische dieser Lösemittel. Bevorzugtes Lösemittel ist Aceton.

In einer bevorzugten Ausführungsform erfolgt die Herstellung der erfindungsgemäßen Verbindungen so, daß zu der acetonischen Lösung des IPDI-Uretdions bei Raumtemperatur das di-sekundäre Diamin so zudosiert wird, daß die Temperatur der Reaktionslösung nicht über 40 °C steigt. Nach Beendigung der Diaminzugabe ist die Reaktion beendet. Es wird das Aceton abdestilliert, das ist dann der Fall, wenn das Reaktionsprodukt noch freie NCO-Gruppen enthalten soll. Soll jedoch das Reaktionsprodukt keine freien NCO-Gruppen mehr enthalten, schließt sich die Umsetzung mit dem Blockierungsmittel bei ca. 60 °C an. Wenn als Blockierungsmittel Alkohole eingesetzt werden, hat es sich als zweckmäßig erwiesen, IPDI-Uretdion bei ca. 70 °C mit Alkoholen lösemittelfrei umzusetzen und nach erfolgter OH/NCO-Reaktion bei Raumtemperatur die Umsetzung mit dem di-sekundären Diamin in Aceton durchzuführen.

Wird auf Lösemittel verzichtet, findet die Umsetzung kontinuierlich in einem Intensivkneter statt, wobei bevorzugt ein Zweiwellenextruder eingesetzt wird.

Ein weiterer Gegenstand der vorliegenden Erfindung ist die Verwendung der erfindungsgemäßen Verbindungen zur Herstellung von Polyurethan-Pulverlacken. Die erfindungsgemäßen Polyurethan-Pulverlacksysteme bestehen aus den erfindungsgemäßen uretdiongruppenhaltigen Polyadditionsverbindungen in Kombination mit hydroxylgruppenhaltigen Polymeren.

Als Polyolkomponente zur Herstellung der erfindungsgemäßen PUR-Pulverlacke kommen grundsätzlich alle OH-Gruppen enthaltenden Polymere in Frage, wie z. B. Epoxidharze, Hydroxyacrylate, bevorzugt werden jedoch hydroxylgruppenhaltige Polyester mit einer OH-Funktionalität von 2 - 5, bevorzugt 3 - 4,2, ein mittleres Molgewicht von 1800 - 5000, bevorzugt 2300 - 4500, einer OH-Zahl von 25 - 120 mg KOH/g, bevorzugt 30 - 90 mg KOH/g, und einem Schmelzpunkt von ≥ 70 bis ≤ 120 °C, bevorzugt ≥ 75 bis ≤ 100 °C.

Für die Herstellung der Polyester bevorzugte Carbonsäuren können aliphatischer, cycloaliphatischer, aromatischer und/oder heterocyclischer Natur sein und gegebenenfalls durch Halogenatome substituiert und/oder ungesättigt sein. Als Beispiele hierfür seien genannt: Bernstein-, Adipin-, Kork-, Azelain-, Sebacin-, Phthal-, Terephthal-, Isophthal-, Trimellith-, Pyromellith-, Tetrahydrophthal-, Endomethylentetrahydrophthal-, Glutar-, Malein- und Fumarsäure bzw. - soweit zugänglich - deren Anhydride, Terephthalsäuredimethylester, weiterhin cyclische Monocarbonsäuren, wie Benzoesäure, p-tert.-Butylbenzoesäure oder Hexahydrobenzoesäure.

Als mehrwertige Alkohole zur Herstellung der für das erfindungsgemäße Verfahren einzusetzenden Polyester kommen z. B. Ethylenglykol, Propylenglykol-1,2 und -1,3, Butylenglykol-1,4 und -2,3, Hexandiol-1,6, Octandiol-1,8, Neopentylglykol, Cyclohexandiol, Bis-(1,4-hydroxymethyl)-cyclohexan, 2,2-Bis-(4-hydroxycyclohexyl)-propan, 2,2-Bis-[4-(β-hydroxyethoxy)-phenyl]-propan, 2-Methyl-propandiol-1,3, 3-Methyl-pentandiol-1,5, 2,2,4(2,4,4)-Trimethylhexandiol-1,6, Glycerin, Trimethylolpropan, Trimethylolethan, Hexantriol-1,2,6, Butantriol-1,2,4, Tris-(β-hydroxyethyl)iso-cyanurat, Pentaerythrit, Mannit und Sorbit sowie Diethylenglykol, Triethylenglykol, Tetraethylenglykol, Dipropylenglykol, Polypropylenglykole, Polybutylenglykole, Xylylenglykol und Hydroxypivalinsäureneopentylglykolester in Frage.

Die Polyester können auf an sich bekannte Weise durch Kondensation in einer Inertatmosphäre bei Temperaturen von 100 - 260 °C, vorzugsweise 130 - 220 °C, in der Schmelze oder in azeotroper Fahrweise gewonnen werden, wie es z. B. in Methoden der Organischen Chemie (Houben-Weyl), Bd. 14/2, 1 - 5, 21 - 23, 40 - 44, Georg Thieme Verlag, Stuttgart, 1963, beschrieben ist.

Bevorzugte Acrylatharze, welche als OH-Komponenten verwendet werden können, sind Homo- oder Copolymerisate, wobei z. B. folgende Monomere als Ausgangsprodukte gewählt werden können:

Ester der Acryl- und Methacrylsäure mit zweiwertigen, gesättigten, aliphatischen Alkoholen mit 2 - 8 C-Atomen, wie z. B. 2-Hydroxyethylacrylat, 2-Hydroxypropylacrylat, 4-Hydroxybutylacrylat und die entsprechenden Methacrylsäureester.

Als hydroxylgruppenhaltige Polymere können selbstverständlich auch Gemische mehrerer Stoffe eingesetzt werden.

Das Mischungsverhältnis der hydroxylgruppenhaltigen Polymeren und den uretdiongruppenhaltigen Polyisocyanaten wird in der Regel so gewählt, daß auf eine OH-Gruppe 0,6 - 1,2, bevorzugt 0,8 - 1,1, ganz besonders bevorzugt 1,0 NCO-Gruppen kommen.

Als weitere Hilfs- und Zusatzstoffe können Katalysatoren, Pigmente, Füllstoffe und Verlaufsmittel im erfindungsgemäßen Verfahren eingesetzt werden.

Die Isocyanatkomponente wird für die Herstellung von PUR-Pulverlacken mit dem geeigneten hydroxylgruppenhaltigen Polymeren und gegebenenfalls Katalysatoren sowie Pigmenten und üblichen Hilfsmitteln, wie Filllstoffen und Verlaufsmittel, z. B. Siliconöl, Acrylatharzen, gemischt und in der Schmelze homogenisiert. Dies kann in geeigneten Aggregaten, wie z. B. beheizbaren Knetern, vorzugsweise jedoch durch Extrudieren erfolgen, wobei Temperaturobergrenzen von 130 - 140 °C nicht überschritten werden sollten. Die extrudierte Masse wird nach Abkühlen auf Raumtemperatur und nach geeigneter Zerkleinerung zum sprühfertigen Pulver vermahlen. Das Auftragen des sprühfertigen Pulvers auf geeignete Substrate kann nach den bekannten Verfahren, wie z. B. durch elektrostatisches Pulversprühen, Wirbelsintern, elektrostatisches Wirbelsintern, erfolgen. Nach dem Pulverauftrag werden die beschichteten Werkstücke zur Aushärtung 60 bis 4 Minuten auf eine Temperatur von 150 bis 220 °C, vorzugsweise 30 bis 6 Minuten bei 160 bis 200 °C, erhitzt.

Nachfolgend wird der Gegenstand der Erfindung anhand von Beispielen näher erläutert.

### Experimenteller Teil

### A) Herstellung der Ausgangsverbindungen für die erfindungsgemäßen Verbindungen

### 1. NCO-Komponente

Als NCO-Komponente kommt das IPDI-Uretdion, wie es in der DE-OS 37 39 549 beschrieben wird, zum Einsatz. Es wird in zwei Stufen hergestellt, wobei in der 1. Stufe IPDI mit 1 % 4-Dimethylaminopyridin ca. 72 h bei Raumtemperatur stehengelassen wird. Danach erfolgt ohne vorherige Desaktivierung des Katalysators die Abtrennung des nicht umgesetzten IPDI mit dem Katalysator durch Dünnschichtdestillation bei 120 °C/0,1 mbar. Der Rückstand enthält einen Gehalt an freiem NCO von 17 - 18 % und 0,5 - 1 % freies IPDI; der Restkatalysatorgehalt beträgt 0,01 - 0,1 %.

### 2. Herstellung des di-sekundären Diamins

### Allgemeine Herstellungsvorschrift

Die Herstellung der N.N'-disubstituierten Diamine erfolgt in zwei Stufen:

In der 1. Stufe wird unter intensivem Rühren der Aldehyd oder das Keton so zum Diamin (Molverhältnis 2:1) zugetropft, daß die Temperatur des Reaktionsgemisches nicht über 40 °C steigt. Nach erfolgter Aldehyd- bzw. Ketonzugabe wird noch 1 h lang bei 40 °C gerührt. Anschließend wird die wäßrige Phase von der organischen abgetrennt. Zur Abtrennung des Restwassers wird die organische Phase bei 60 °C und 10 mbar so lange erhitzt, bis kein Wasser mehr übergeht. In der 2. Stufe erfolgt die Hydrierung der Dischiffschen Base an einem Co-Kontakt bei 125 °C und 300 at. Anschließend wird das Rohprodukt destilliert.

| **Ausgangsverbindungen** | | | **Reaktionsprodukt** | |
|---|---|---|---|---|
| **Beispiel Nr.** | **Diamin** | **CO-Komponente** | **Amingehalt [mmol/g]** | **Reinheit (GC)** |
| 1 | TMD | Diisobutylketon | 4,9 | > 98 |
| 2 | TMD | Methyl-isobutylketon | 6,4 | > 99 |
| 3 | IPD | i-Butyraldehyd | 7,1 | > 98 |
| 4 | IPD | Methyl-isobutylketon | 5,9 | > 98 |

### B: Herstellung der erfindungsgemäßen Verbindungen

### Allgemeine Herstellungvorschrift

Zu der acetonischen Lösung des IPDI-Uretdions wird bei Raumtemperatur das di-sekundäre Diamin unter intensiver Rührung so zudosiert, daß die Temperatur der Reaktionslösung 40 °C nicht übersteigt. Nach Beendigung der Diaminzugabe ist die Reaktion praktisch beendet, und es erfolgt die Acetonentfernung. Sollen die freien NCO-Gruppen des IPDI-Uretdion/Diamin-Additionsproduktes (Molverhältnis: IPDI-Uretdion: Diamin = (n+1):n) mit Oximen oder sekundären Monoaminen blockiert werden, so geht man so vor, daß nach der IPDI-Uretdion/Diamin-Reaktion das Blockierungsmittel bei ca. 40 °C portionsweise zudosiert und ca. 1 h bei 60 °C weiter erhitzt wird. Danach erfolgt die Acetonentfernung. Sollen die freien NCO-Gruppen des IPDI-Uretdion/Diamin-Additionsproduktes mit Alkoholen (HO-R⁵) blockiert werden, so hat sich folgende Verfahrensweise als vorteilhaft erwiesen:
a) Umsetzung des IPDI-Uretdions mit dem Alkohol in Substanz bei ca. 70 °C (0,5 h),
b) anschließende Abkühlung und Lösung in Aceton,
c) Umsetzung dieser acetonischen Lösung mit den diskundären Diaminen.

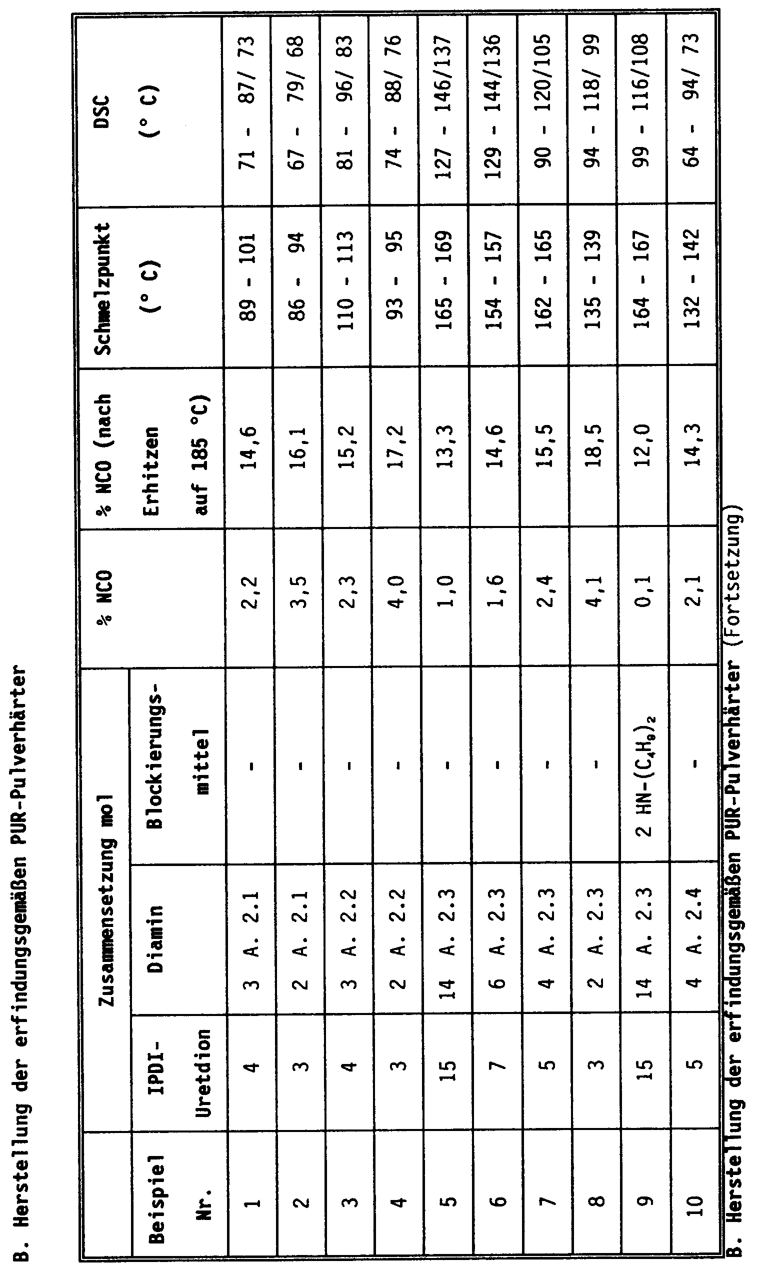

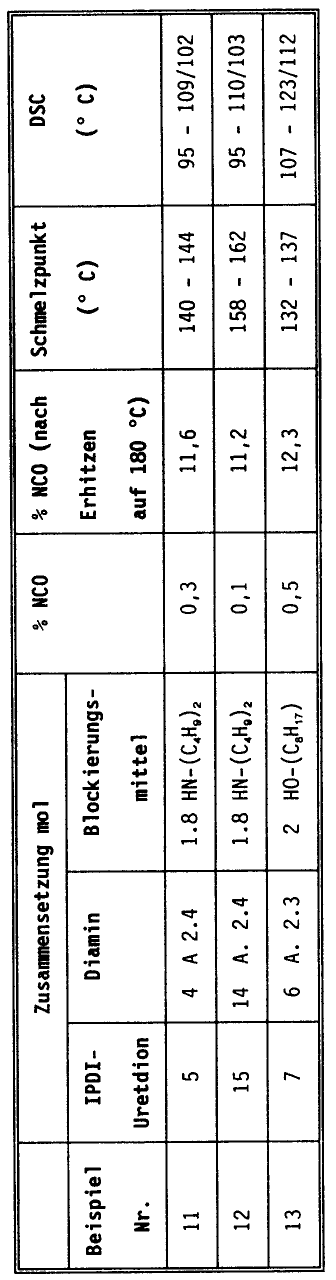

### C) Polyurethan-Pulverlacke

### C) 1. Polyolkomponente

Zur Herstellung der unter C) 2 beschriebenen Pulverlacke wurde als Polyolkomponente der Oxyester ALFTALAT AN 739 der Fa. Hoechst mit folgenden Kenndaten eingesetzt:
- OH-Zahl:: 55 - 60 mg KOH/g
- Säurezahl:: 2 - 4
- Schmelzbereich:: 82 - 90 °C
- Glasumwandlungstemperatur:: > 50 °C
- Viskosität (bei 160 °C):: 24 - 29 000 mPa·s

### C) 2. Herstellung der PUR-Pulverlacke - Allgemeine Herstellungsvorschrift

Die zerkleinerten Produkte, Vernetzer (B), Polyester C) 1., Verlaufsmittel-, Katalysator-Masterbatch, werden mit dem Weißpigment in einem Kollergang innig vermischt und anschließend im Extruder bis maximal 130 °C homogenisiert. Nach dem Erkalten wird das Extrudat gebrochen und mit einer Stiftmühle auf eine Korngröße < 100 µm gemahlen. Das so hergestellte Pulver wird mit einer elektrostatischen Pulverspritzanlage bei 60 KV auf entfettete, ggf. vorbehandelte Eisenbleche appliziert und in einem Umlufttrockenschrank bei Temperaturen zwischen 180 und 200 °C eingebrannt.

### Verlaufsmittel-Masterbatch

Es werden 10 Gew.-% des Verlaufsmittels - ein handelsübliches Copolymer von Butylacrylat und 2-Ethylhexylacrylat - in dem Polyester C) 1 in der Schmelze homogenisiert und nach dem Erstarren zerkleinert.

### Katalysator-Masterbatch

Es werden 5 Gew.-% des Katalysators - Dibutylzinndilaurat (DBTL) -im Polyester C) 1 in der Schmelze homogenisiert und nach dem Erstarren zerkleinert.

Die in der nachfolgenden Tabelle angegebenen PUR-Pulverlacke enthalten 40 Gew.-% TiO₂ (Weißpigment) sowie je 0,5 Gew.-% Verlaufsmittel und Benzoin sowie 0,15 Gew.-% DBTL. Das OH:NCO-Verhältnis beträgt 1:1.

Die Abkürzungen in der folgenden Tabelle bedeuten:
- SD: = Schichtdicke in µm
- HK: = Härte nach König (DIN 53 157)
- HB: = Härte nach Buchholz (DIN 53 153)
- ET: = Tiefung nach Erichsen (DIN 53 156)
- GG 60° ∢: = Messung des Glanzes nach Gardner (ASTM-D 5233)
- KS d: = Kugelschlag direkt
- KS i: = Kugelschlag indirekt

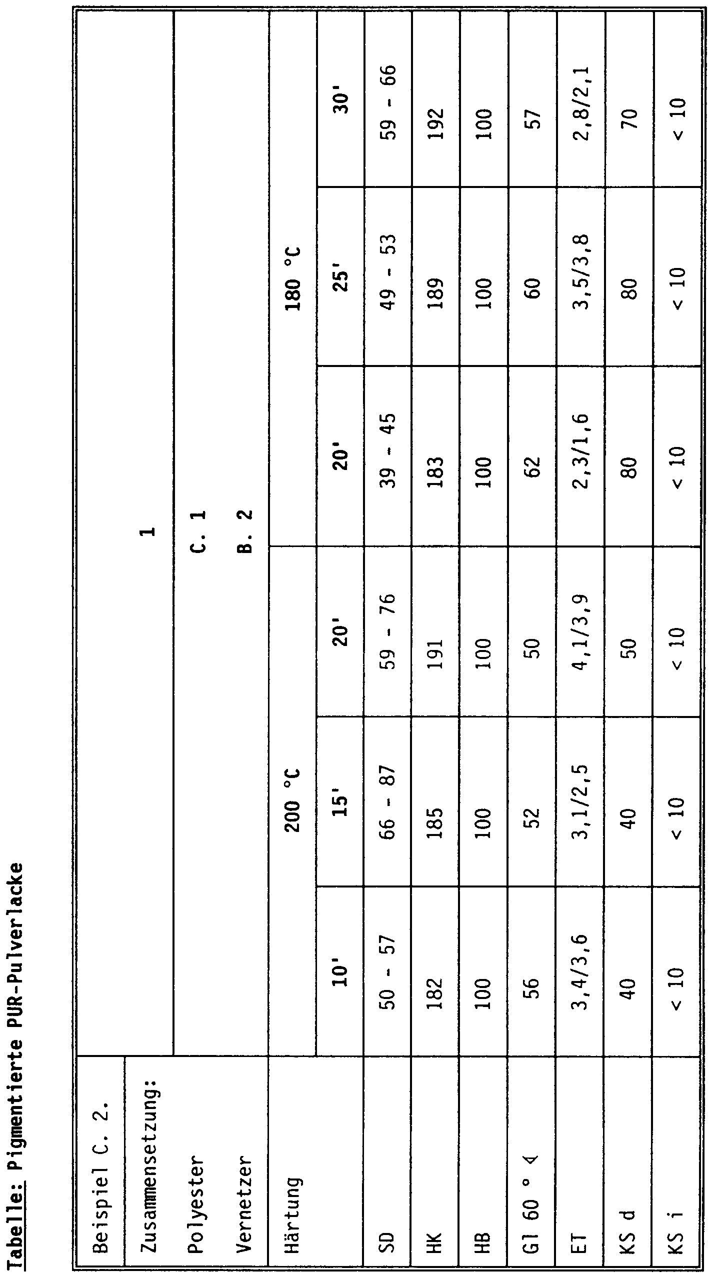

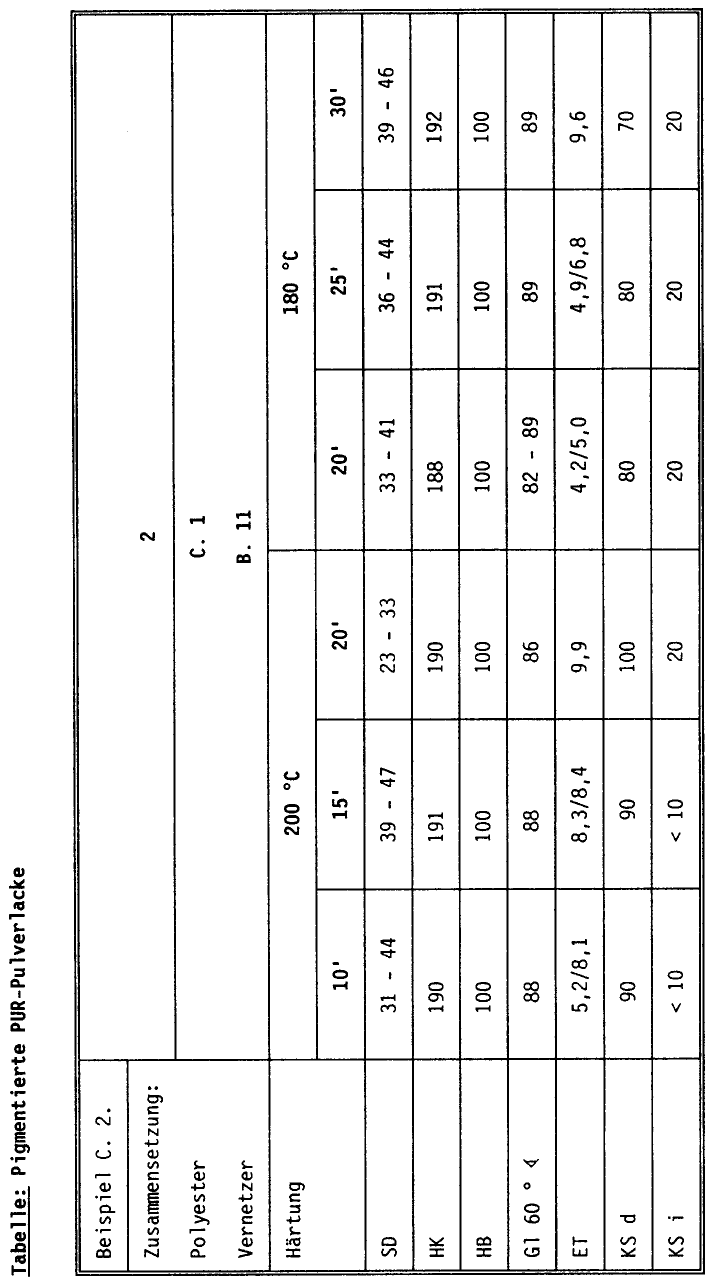

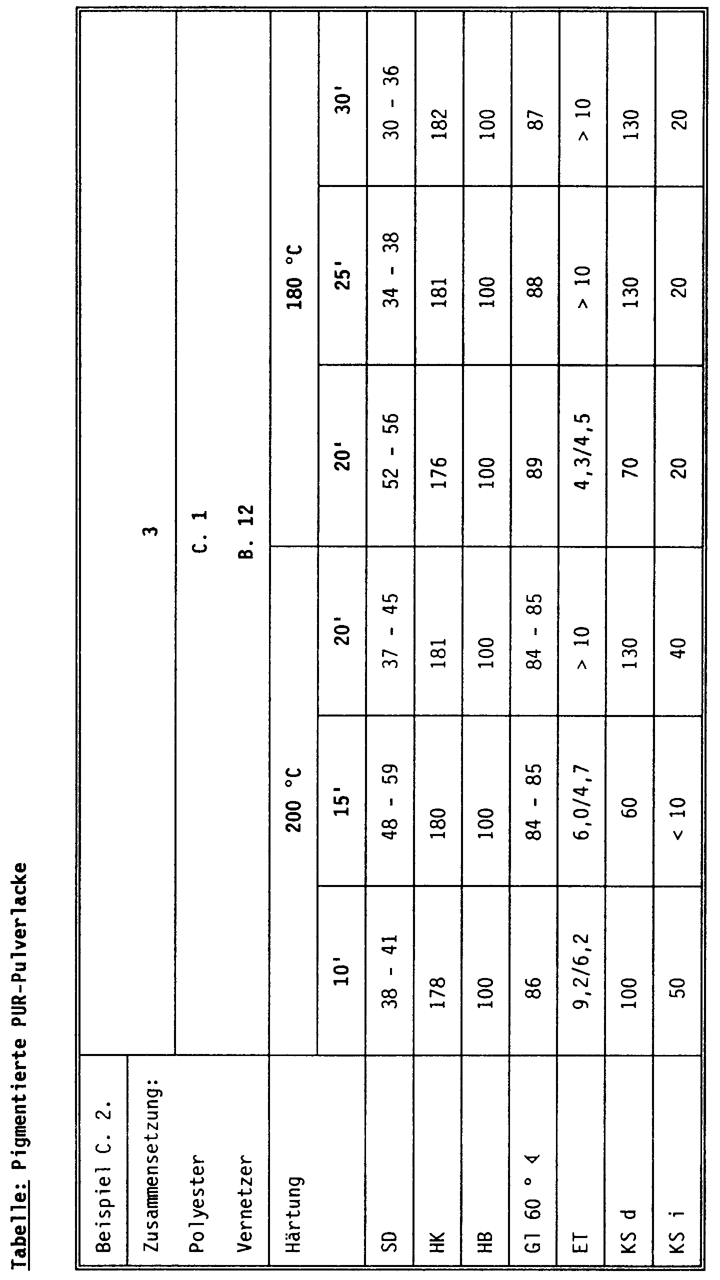

## Patentansprüche

1. Uretdiongruppenhaltige Polyadditionsprodukte der allgemeinen Formel I, erhältlich durch Reaktion des Isophorondiisocyanat-Uretdions (IPDI-Uretdion) mit di-sekundären Diaminen 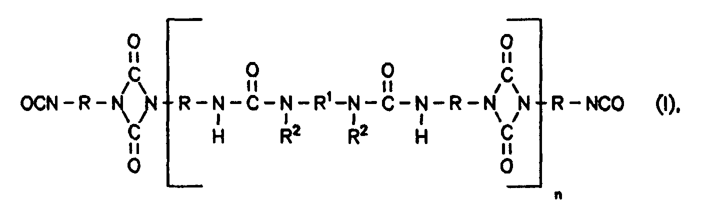 wobei
R der Formel II entspricht 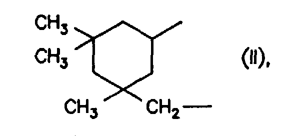 und
R¹ gleich R oder einen cycloaliphatischen oder aliphatischen, linearen oder verzweigten Alkylenrest mit 2 - 16 C-Atomen,
R² einen cycloaliphatischen oder aliphatischen Kohlenwasserstoffrest mit 1 - 12 C-Atomen oder einen Rest der Formel III 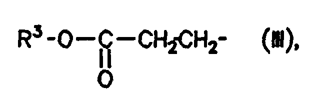
R³, R⁴, und R⁵ gleiche oder verschiedene Kohlenwasserstoffreste mit 1 -14 C-Atomen und n gleich 1 - 15 bedeuten.

2. Uretdiongruppenhaltige Polyadditionsprodukte nach Anspruch 1,
dadurch gekennzeichnet,
daß die NCO-Gruppen teilweise oder vollständig mit Blockierungsmitteln blockiert sind.

3. Uretdiongruppenhaltige Polyadditionsprodukte nach den Ansprüchen 1 bis 2,
dadurch gekennzeichnet,
daß sie reversibel oder irreversibel blockiert sind.

4. Uretdiongruppenhaltige Polyadditionsprodukte nach Anspruch 3,
dadurch gekennzeichnet,
daß als reversibles Blockierungsmittel Ketoxime eingesetzt werden.

5. Uretdiongruppenhaltige Polyadditionsprodukte nach Anspruch 4,
dadurch gekennzeichnet,
daß Acetonoxim, Methylethylketoxim, Acetophenonoxim oder Cyclohexanonoxim, eingesetzt werden.

6. Uretdiongruppenhaltige Polyadditionsprodukte nach Anspruch 3,
dadurch gekennzeichnet,
daß als irreversible Blockierungsmittel Monoalkohole und/oder sekundäre Amine eingesetzt werden.

7. Uretdiongruppenhaltige Polyadditionsprodukte nach Anspruch 6,
dadurch gekennzeichnet,
daß Methanol, Ethanol, Butanol, 2-Ethylhexanol, Dibutylamin, Di-2-ethylhexylamin oder Methylcyclohexylamin eingesetzt werden.

8. Uretdiongruppenhaltige Polyadditionsprodukte nach mindestens einem der Ansprüche 1 bis 7,
dadurch gekennzeichnet,
daß als di-sekundäre Diamine solche eingesetzt werden, die durch Kondensation von di-primären Diaminen mit Carbonylverbindungen zur Schiffschen Base und anschließender Hydrierung, oder durch Reaktion von di-primären Diaminen mit Acrylsäureestern, erhalten werden.

9. Uretdiongruppenhaltige Polyadditionsprodukte nach Anspruch 8,
dadurch gekennzeichnet,
daß als di-primäre Diamine Ethylendiamin, 1,2-Diaminopropan, 2-Methyl-pentamethylendiamin, Hexamethylendiamin, 2,2,4(2,4,4)-Trimethylhexamethylendiamin, Isophorondiamin (IPD), 1,2-Diaminocyclohexan oder 1,3-Bis(aminomethyl)benzol eingesetzt werden.

10. Uretdiongruppenhaltige Polyadditionsprodukte nach Anspruch 8,
dadurch gekennzeichnet,
daß als Carbonylverbindung Aldehyde und Ketone eingesetzt werden.

11. Uretdiongruppenhaltige Polyadditionsprodukte nach Anspruch 10,
dadurch gekennzeichnet,
daß Isobutyraldehyd, 2-Ethylhexanal, Methylethylketon, Methylisobutylketon, Diisobutylketon, Cyclohexanon oder 3,5,5-Trimethylcyclohexanon eingesetzt werden.

12. Verfahren zur Herstellung der Verbindungen gemäß den Ansprüchen 1 bis 11,
dadurch gekennzeichnet,
daß n+1 mol IPDI-Uretdion mit n mol di-sekundärem Diamin zur Reaktion gebracht werden und dabei die Temperatur nicht über 40 °C steigt, wobei n gleich 1 - 15 betragen kann.

13. Verfahren nach Anspruch 12,
dadurch gekennzeichnet,
daß die freien NCO-Gruppen vor oder nach der Reaktion mit den di-sekundären Diaminen mit Blockierungsmitteln zur Reaktion gebracht werden.

14. Verwendung der Verbindungen gemäß den Ansprüchen 1 bis 11 als Vernetzerkomponente zur Herstellung von Polyurethan-Pulverlacken.
